# EUROPEAN PATENT APPLICATION

(11) **EP 4 349 364 A1**
(43) Date of publication of application: **10.04.2024**
(21) Application number: 22816408.3
(22) Date of filing: 30.05.2022
(51) Int. Cl.: A61K 38/17, A61P 9/00, A23L 33/17

(54) **COMPOSITION FOR PREVENTING OR TREATING VASCULAR DISEASES, CONTAINING HAPLN1**

(30) Priority: 31.05.2021 KR 20210069822
(71) Applicant: Chung Ang University Industry Academic Cooperation Foundation, Seoul 06974 (KR)
(72) Inventor: KIM, Dae Kyong, Seongnam-si Gyeonggi-do 13547 (KR)
(74) Representative: Roos, Peter
(86) International application number: PCT/KR2022/007649
(87) International publication number: WO 2022/255749

(57) **Abstract**

The present invention relates to a composition for repairing vascular degeneration and vascular injuries and preventing or treating vascular diseases, containing hyaluronan and proteoglycan link protein 1 (HAPLN1) as an active ingredient. More specifically, provided is a pharmaceutical composition and a health functional food composition for preventing or treating vascular diseases, containing HAPLN1 as an active ingredient. HAPLN1 inhibits and alleviates, in blood vessel walls, damage to the elastin fiber layer and loss of transgelin, and effectively inhibits the phosphorylation of NF-κB or focal adhesion kinase (FAK) in vascular smooth muscle cells (VSMCs), and thus can be used for preventing, alleviating or treating vascular injuries and various diseases caused by aging or a high-fat diet.

## Description

### Technical Field

The present disclosure relates to a composition for repairing vascular degeneration and damaged blood vessels and preventing or treating vascular diseases, including hyaluronan and proteoglycan link protein 1 (HAPLN1) as an active ingredient.

### Background Art

Blood vessels are large and small vessels that exist in the body of an animal, responsible for pumping out blood that carries oxygen and nutrients to every part of the body and taking in carbon dioxide and waste products excreted by peripheral cells and tissues. Blood vessels are divided into arteries, veins, and capillaries based on their structure and function. Arteries are vessels that send blood bearing oxygen or nutrients from the heart to the rest of the body and gradually taper toward capillaries. Exchange of substances takes place between capillaries and tissues. The capillaries converge to become a vein that runs back to the heart. The total length of human blood vessels is about 120,000 km.

With global acceleration in the aging, interest in healthy longevity is increasing, and the demand for prevention and treatment of age-related geriatric and chronic diseases is rising as well. It has been found that the main cause of death in 8 out of 10 deaths in Korea is the chronic disease such as cancer, cardiovascular/cerebrovascular diseases, diabetes, and chronic respiratory diseases, among which a vascular disease is the second leading cause of death in Korea while it is the first cause of death in the world. These vascular diseases increase even more with the aging of the population, with a sudden increase after the age of 50 to the maximum at the age of 75-79, which is the average life expectancy of Koreans, and the disease remains at a relatively high level until the age of 85. Vascular diseases are closely related to aging, as in the saying that "people grow old with blood vessels" or "old blood vessels are the soil that nurtures the seeds of cardiovascular/cerebrovascular diseases".

Vascular disease is a disease in which blood vessels are blocked or burst and lead to impaired supply of blood to tissues to cause arteriosclerosis, aneurysm, high blood pressure/hemorrhage, ischemia, and vascular wall disorders, thereby resulting in various diseases such as cerebral infarction, cerebral hemorrhage, ischemic heart disease, myocardial infarction, and hypertension. Major vascular diseases include arteriosclerosis and hypertension, of which arteriosclerosis is a disease that cholesterol accumulates inside the blood vessels to narrow or block the blood vessels, just as foreign substances accumulate in old water pipes to cause blocking, and is also a chronic inflammatory cardiovascular disease that causes angina or myocardial infarction and, in severe cases, sudden death.

According to Baltimore Longitudinal Study of Aging (BLSA) established in 1958 by the National Institutes of Health (NIH), the main transition in blood vessels that occur with age is called 'blood vessel aging' or 'vascular aging', and the main characteristics are thickened vascular walls, decreased elasticity, and vascular endothelial cell dysfunction, suggesting these structural and functional changes as the ultimate major factors of atherosclerosis and hypertension. In 2003 study in Science, Dr. Martin of the University of Washington related these vascular diseases to aging and emphasized the importance of prevention rather than treatment as well as aging delay in order to overcome the disease. Therefore, in order to cope with the vascular disease, research to develop technologies or drugs that may delay or prevent vascular aging is very crucial. In addition, in 2016, Peter et al. emphasized the need for prevention of cardiovascular diseases and proposed early vascular aging as a key concept.

The discovery and development of drugs that may prevent or reverse vascular media thickness and elasticity loss, which are major changes in vascular aging, are known to be the key for overcoming vascular-related diseases, but no such drug has emerged to date. The cells that play a pivotal role in these pathological changes are vascular smooth muscle cells (VSMCs). VSMC, which is most abundant in the wall of normal blood vessels, especially in the media, is a type of muscle cells that play a central role in the contractility and distensibility of blood vessels and primarily involved in formation of vascular lesions.

In normal blood vessels, VSMCs produce elastin fibers, which are polymers of elastin, a major protein necessary for contractility and distensibility, and they differentiate into cells that are rich in transgelin (SM22α, TAGLN), a biomarker of healthy normal cells, and cease to proliferate at the same time. This type of cell is called a contractile phenotype of VSMC. However, when chronic abnormalities occur in the vascular function due to aging or degeneration and the cells are converted to a de-differentiated state and return to the pre-differentiation stage, the cells are switched to synthetic or proliferative phenotype of VSMC and resume proliferation and migration which becomes a starting point for the development of arteriosclerosis, and this pathological change is well known as the main cause of transgelin loss, which is a particularly key biomarker. As a result of these biochemical changes, the blood vessel wall at the lesion site thickens and hardens, and at the same time, various monocytes and other inflammatory cells are involved and activated, resulting in various vascular diseases such as atherosclerosis, hypertension, aneurysm, and restenosis after undergoing a series of processes.

Looking over the existing technologies related to the HAPLN1 protein, they propose its use and role as a biomarker by measuring a level of the protein or report the protein as a substance that has the potential to cause diseases rather than to inhibit vascular diseases. Therefore, there are no reports that suggest the potential of HAPLN1 as a drug capable of treating or preventing related diseases by suppressing vascular diseases such as hypertension and arteriosclerosis.

### Disclosure of the Invention

### Technical Goals

An object of the present disclosure is to provide a composition for treating a vascular disease using a protein that has an effect of inhibiting vascular aging and damage. Technical Solutions

In order to achieve the above object, the present disclosure provides a pharmaceutical composition for preventing or treating a vascular disease, including hyaluronan and proteoglycan link protein 1 (HAPLN1) as an active ingredient.

In addition, the present disclosure provides a health functional food composition for preventing or alleviating a vascular disease, including HAPLN1 as an active ingredient. Advantageous Effects

HAPLN1 according to the present disclosure inhibits and alleviates damage in elastic lamina and loss of transgelin in the vascular wall and effectively suppresses phosphorylation of NF-κB or focal adhesion kinase (FAK) in vascular smooth muscle cells (VSMCs), and thus may be utilized as a pharmaceutical composition or health functional food composition for preventing or treating vascular damage and various vascular diseases due to aging or high-fat diet.

### Brief Description of Drawings

FIG. 1 shows changes in the thoracic artery wall depending on whether human recombinant HAPLN1 (rhHAPLN1) is administered in high-fat diet mice according to an experimental example of the present disclosure.
FIG. 2 shows results of elastic tissue fiber-Verhoeffs Van Gieson (EVG) staining capable of quantitatively measurement of a level in order to observe changes in an elastin fiber content depending on rhHAPLN1 administration in high-fat diet mice.
FIG. 3 shows results of identifying an elastin fiber recovery effect in the thoracic aorta of high-fat diet mice using Victoria-blue van Gieson staining and elastin degradation processes to evaluate statistical significance.
FIG. 4 shows a result of identifying an elastin recovery effect according to an evaluation criterion for an elastin degradation process (** P <0.01).
FIG. 5 shows results of staining using a red transgelin antibody labeled with a fluorescent substance (anti-transgelin antibody labeled with Alexa Fluor) to observe quantitative changes in transgelin, a key marker of the atherosclerotic arterial vessel wall.
FIG. 6 shows a result of measuring a red area (transgelin+ area) in a transgelin region in FIG. 5 using Image J software.
FIG. 7 shows results of identifying changes in phosphorylated NF-κB (p-NF-κB) upon administration of rhHAPLN1 in human aortic smooth muscle cells (HASMCs) treated with interleukin-1 beta (IL-1β) known as an inducer of vascular aging, in which five independent experiments were conducted, with similar results derived each time and which showed representative results.
FIG. 8 shows results of identifying changes in phosphorylated focal adhesion kinase (p-FAK) upon administration of rhHAPLN1 in HASMC treated with IL-1β as in FIG. 7, in which five independent experiments were conducted, with similar results derived each time and which showed representative results.

### Best Mode for Carrying Out the Invention

Hereinafter, the present disclosure will be described in more detail.

The present inventors completed the present disclosure by determining that a HAPLN1 protein may treat a vascular disease through various mechanisms.

The present disclosure provides a pharmaceutical composition for preventing or treating a vascular disease, including hyaluronan and proteoglycan link protein 1 (HAPLN1) as an active ingredient.

As used herein, the term "HAPLN1" refers to a protein that stabilizes hyaluronic acid by linking hyaluronic acid to proteoglycan and a constituent protein in the extracellular matrix that was first discovered in the joints of vertebrates.

Preferably, the HAPLN1 may be a protein fragment having an amino acid sequence represented by SEQ ID NO: 1 and may be a recombinant human HAPLN1 (rhHAPLN1).

SEQ ID NO: 1:

DHLSDNYTLDHDRAIHIQAENGPHLLVEAEQAKVFSHRGGNVTLPCKFYRDPTAFGS
GIHKIRIKWTKLTSDYLKEVDVFVSMGYHKKTYGGYQGRVFLKGGSDSDASLVITD
LTLEDYGRYKCEVIEGLEDDTVVVALDLQGVVFPYFPRLGRYNLNFHEAQQACLDQ
DAVIASFDQLYDAWRGGLDWCNAGWLSDGSVQYPITKPREPCGGQNTVPGVRNYG
FWDKDKSRYDVFCFTSNFNGRFYYLIHPTKLTYDEAVQACLNDGAQIAKVGQIFAA
WKILGYDRCDAGWLADGSVRYPISRPRRRCSPTEAAVRFVGFPDKKHKLYGVYCFR
AYN

In the present disclosure, the composition may prevent or treat vascular damage or various vascular diseases caused by aging or high-fat diet.

As used herein, the term "prevention" refers to any action of inhibiting or delaying the onset of a vascular disease or at least one or more symptoms of the disease by administration of the pharmaceutical composition or health functional food composition according to the present disclosure. In addition, it includes treatment of subjects who are in remission of the disease in order to prevent or avoid recurrence.

As used herein, the term "treatment" refers to any action of ameliorating or beneficially changing the symptoms such as by alleviating, reducing, or eliminating a vascular disease, or at least one or more symptoms of the disease by the administration of the pharmaceutical composition according to the present disclosure.

More specifically, the composition may inhibit and alleviate the damage in elastic lamina of a vascular wall and inhibit and ameliorate the loss of transgelin. In addition, the composition may suppress phosphorylation of NF-κB or focal adhesion kinase (FAK) in vascular smooth muscle cells (VSMCs).

According to an example embodiment of the present disclosure, it was found that an arterial vascular wall of a mouse damaged by high-fat diet was restored to a shape almost similar to that of the arterial vessel wall of a normal diet mouse by the administration of the HAPLN1, the area of reduced transgelin was increased, and phosphorylation of NF-κB and FAK was suppressed in human aortic smooth muscle cells, which is a type of vascular smooth muscle cell.

The composition with this effect may be used for preventing or treating vascular diseases, including vascular damage caused by aging or high-fat diet, and for example, the vascular disease may be one or more selected from the group consisting of, but is not limited to, arteriosclerosis, hypertension, aneurysm, hemorrhage, cerebral infarction due to vascular wall obstruction, cerebral hemorrhage, ischemic heart disease, myocardial infarction, and peripheral vascular disease.

The pharmaceutical composition according to the present disclosure may be prepared according to the conventional methods in the pharmaceutical field. The pharmaceutical composition may be blended with a pharmaceutically acceptable, appropriate carrier in accordance with the formulation in addition to the active ingredient and, as required, may be prepared by further including excipients, diluents, dispersants, emulsifiers, buffers, stabilizers, binders, disintegrators, and solvents. The appropriate carrier does not inhibit the activity and characteristics of HAPLN1 according to the present disclosure and may be selected depending on the dosage type and formulation.

The pharmaceutical composition may be applied in any formulation, and more specifically, it may be formulated and used in the form of oral formulations, topical agents, suppositories, and parenteral formulations of a sterile injection solution according to the conventional method.

Of the oral formulations, the solid formulation is in the form of tablets, pills, acids, granules, and capsules, wherein at least one or more excipients, for example, starch, calcium carbonate, sucrose, lactose, sorbitol, mannitol, cellulose, and gelatin may be mixed for preparation, and lubricants such as magnesium stearate and talc may also be included in addition to the simple excipients. In addition, in the case of the capsule formulation, it may further include liquid carriers such as fatty oils in addition to the substances mentioned above.

Of the oral formulations, the liquid formulation may include suspensions, solutions, emulsions, and syrups, and various excipients such as wetting agents, sweeteners, fragrances, and preservatives may be included in addition to commonly used simple diluents such as water and liquid paraffin.

The parenteral formulation may include sterile aqueous solutions, non-aqueous solutions, suspensions, emulsions, freeze-dried preparations, and suppositories. Propylene glycol, polyethylene glycol, vegetable oils such as olive oil, and injectable esters such as ethyl oleate may be used as non-aqueous solvents and suspensions. Witepsol, macrogol, Tween 61, cacao butter, laurin fat, and glycerogelatin may be used as a base of the suppositories. All suitable preparations known in the art may be used without limitation.

In addition, the pharmaceutical composition according to the present disclosure may be added with calcium or vitamin D₃ to enhance a therapeutic efficacy.

The pharmaceutical composition according to the present disclosure may be administered in a pharmaceutically effective amount.

As used herein, the term "pharmaceutically effective amount" refers to an amount sufficient to treat the disease at a reasonable benefit/risk ratio that is applicable to medical treatment without causing adverse effects.

The effective dose level of the pharmaceutical composition may depend on the purpose of use, the patient's age, sex, weight and health status, type of disease, severity, activity of the drug, sensitivity to the drug, method of administration, time of administration, route of administration and discharge rate, treatment period, factors including drugs that are blended or concurrently used, and other factors well known in the field of medicine. For example, it may be administered inconsistently, but generally once or several times daily in an amount of 0.001 to 100 mg/kg, preferably 0.01 to 10 mg/kg. The above dosage is not intended to limit the scope of the present disclosure in any way.

The pharmaceutical composition may be administered to any animal in which vascular disease may develop, and the animal may include, for example, humans and primates, as well as domestic animals such as cattle, pigs, horses, and dogs.

The pharmaceutical composition may be administered by a suitable route of administration according to the preparation form and, as long as it may reach the target tissue, it may be administered through various routes, either orally or parenterally. The method of administration may be administered by conventional methods, including, but not limited to, for example, oral, rectal or intravenous, intramuscular, skin application, subcutaneous, intrarespiratory inhalation, intrauterine dural, or intracerebroventricular injection.

The pharmaceutical composition may be used alone for preventing or treating vascular diseases, or in combination with surgery or other drug treatment.

In addition, the present disclosure provides a health functional food composition for preventing or alleviating a vascular disease, including hyaluronan and proteoglycan link protein 1 (HAPLN1) as an active ingredient.

Preferably, the HAPLN1 may be a protein fragment having an amino acid sequence represented by SEQ ID NO: 1 and may be a recombinant human HAPLN1 (rhHAPLN1).

The composition may inhibit and alleviate damage to the elastic lamina in the vascular wall, and inhibit and ameliorate the loss of transgelin. In addition, the composition may suppress phosphorylation of NF-κB or focal adhesion kinase (FAK) in vascular smooth muscle cells (VSMCs), thereby preventing or alleviating vascular damage or various vascular diseases caused by aging or high-fat diet.

As used herein, the term "alleviation" refers to any action of ameliorating or beneficially changing the symptoms such as by alleviating, reducing, or eliminating a vascular disease, or at least one or more symptoms of the disease by consumption of the health functional food composition according to the present disclosure.

The vascular disease may be one or more selected from the group consisting of, but is not limited to, arteriosclerosis, hypertension, aneurysm, hemorrhage, cerebral infarction due to vascular wall obstruction, cerebral hemorrhage, ischemic heart disease, myocardial infarction, and peripheral vascular disease.

In the health functional food composition according to the present disclosure, the health functional food may be prepared in the form of powder, granules, tablets, capsules, syrups, or beverages for the purpose of preventing or alleviating vascular diseases, wherein there is no limitation in the form that the food may take, and all foods in the ordinary sense may be included. For example, it may include beverages and various drinks, fruits and processed foods thereof (canned fruits, jams, etc.), fish, meat and processed foods thereof (ham, bacon, etc.), breads and noodles, cookies and snacks, and dairy products (butter, cheese, etc.), and all functional foods in the usual sense may be included. It may also include foods that are used as feed for animals.

The health functional food composition may be prepared by further including additional foodologically acceptable food additives (food additives) and other appropriate supplementary ingredients commonly used in the art.

The suitability of the food additives may be determined by the standards and criteria related to corresponding items according to the general rules and general test methods of Korean Food Additives Codex approved by the Ministry of Food and Drug Safety, unless otherwise stipulated. The items listed in the "Korean Food Additives Codex" may include, for example, chemically synthesized compounds such as ketones, glycine, potassium citrate, nicotinic acid, and cinnamic acid; natural additives such as persimmon color, licorice extracts, crystallized cellulose, kaoliang color, and guar gum; and mixed preparations such as sodium L-glutamate preparations, noodle-added alkali agents, preservative agents, and tar color agents.

The other supplementary ingredients may further include, for example, flavors, natural carbohydrates, sweeteners, vitamins, electrolytes, colorants, pectic acids, alginic acids, organic acids, protective colloidal thickeners, pH regulators, stabilizers, preservatives, glycerin, alcohols, and carbonators. In particular, monosaccharides such as glucose and fructose, disaccharides such as maltose and sucrose, and polysaccharides such as dextrin and cyclodextrin, sugar alcohols such as xylitol, sorbitol, and erythritol may be used as the natural carbohydrates, and natural sweeteners such as thaumatin and stevia extract or synthetic sweeteners such as saccharin and aspartame may be used as sweeteners.

The effective dose of HAPLN1 included in the health functional food according to the present disclosure may be adjusted appropriately according to the purpose of use, such as the prevention or alleviation of the vascular disease. The composition has the advantage of not causing side effects that may occur in the long-term intake of general drugs by using food as a raw material, while securing excellent portability, such that it may be taken as a supplement for preventing or alleviating vascular diseases.

### Modes for Carrying Out the Invention

Hereinafter, example embodiments will be described in detail to help the understanding of the present disclosure. However, the following example embodiments are merely illustrative of the content of the present disclosure, and the scope of the present disclosure is not limited to the following example embodiments. The example embodiments of the present disclosure are provided to more completely explain the present disclosure to those skilled in the art.

### <Example 1> Production of recombinant human HAPLN1 protein (rhHAPLN1)

### 1-1. Amino acid sequence of recombinant human HAPLN1 protein

The amino acid sequence forming the recombinant HAPLN1 protein is as follows.

DHLSDNYTLDHDRAIHIQAENGPHLLVEAEQAKVFSHRGGNVTLPCKFYRDPTAFGS
GIHKIRIKWTKLTSDYLKEVDVFVSMGYHKKTYGGYQGRVFLKGGSDSDASLVITD

LTLEDYGRYKCEVIEGLEDDTVVVALDLQGVVFPYFPRLGRYNLNFHEAQQACLDQ
DAVIASFDQLYDAWRGGLDWCNAGWLSDGSVQYPITKPREPCGGQNTVPGVRNYG
FWDKDKSRYDVFCFTSNFNGRFYYLIHPTKLTYDEAVQACLNDGAQIAKVGQIFAA
WKILGYDRCDAGWLADGSVRYPISRPRRRCSPTEAAVRFVGFPDKKHKLYGVYCFR
AYN (SEQ ID NO: 1)

### 1-2. Expression, purification, and storage of recombinant human HAPLN1 protein (rhHAPLN1)

For preparation of recombinant human HAPLN1 (rhHAPLN1; gene number 2678736), a DNA vector encoded with the recombinant human HAPLN1 amino acid sequence was used as the host cell for Expi^{™} 293 cells (ThermoFisher Scientific Co., Waltham, MA, USA). For purification, the amino ends were expressed with a secretion signal peptide, 10 histidines (H, His), and a TEV protease-recognition site inserted. After 3 days of vector infection, the culture medium was collected and purified via HisTrap column (GE Healthcare, IL, USA), the TEV protease-cognitive sequence was cleaved with TEV protease, and the histidine-including molecules were removed using DynaBeads (Thermo Fisher Scientific). The solution obtained thereby was subjected to dialysis for 40 mM Tris-HCl and 1 M NaCl at pH 8.0 for 16 hours. The protein concentration of the finally purified product was 0.11 mg/ml, and the solvent was divided in a single dose with 20 mM Tris-HCl, 0.5 M NaCl, pH 8.0, and 50% glycerol to be stored in a refrigerator at -20°C.

### <Example 2> Identification of arterial vascular structure improving effect in high-fat diet mice by repeated intraperitoneal administration of recombinant human HAPLN1 protein (rhHAPLN1)

### 2-1. Preparation and breeding of laboratory animals

Laboratory animals were 9-week-old female C57BL/6J (Korea Basic Science Research Institute (KBSI), Daejeon, South Korea) mice and set as control groups (PBS) and administration groups (rhHAPLN1). Water was changed once every two days and left for drink freely, 4 mice were placed in one cage and fed with 22 g of high-fat feed every day, the temperature in the breeding room was kept at 21~24°C with 40%~60% humidity, and the cycle of day and night was 12 hours respectively. The control group and the administration group were assigned 8 mice each. The treated group (rhHAPLN1) was subjected to intraperitoneal (IP) injection with recombinant rhHAPLN1 diluted in phosphate buffered saline (PBS) in a dose of 0.1 mg/kg by an amount of 100 µl at a time, and administration was conducted once every three days, 10 times for a total of 30 days. The other two control groups were administered with the same amount of PBS in the same way.

### 2-2. Identification of a vascular structure improving effect of human recombinant HAPLN1 by H&E staining of the artery wall of high-fat diet mice

High-fat diet mice were fed with atherogenic Paigen diet (PD; 15.8% fat: about half cocoa butter, 1.25% cholesterol and 0.5% cholesterol, product number TD88051l) feed for 16 weeks. The high-fat diet mice were injected intraperitoneally with PBS or rhHAPLN1, and after anesthesia in each group of mice, an incision was performed in the abdominal cavity to collect the mouse thoracic artery blood vessels and observe the artery wall.

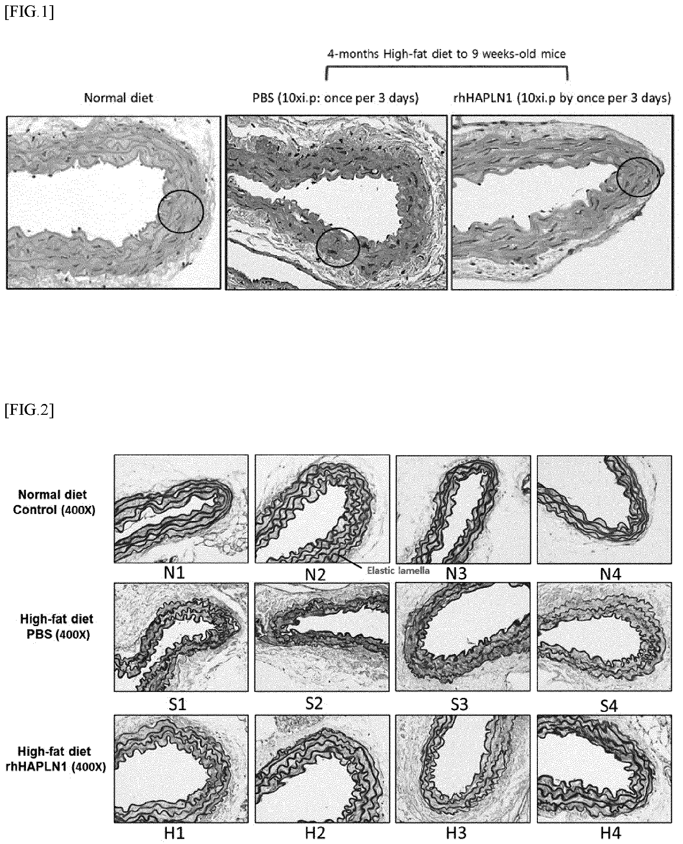

As a result, as shown in FIG. 1, compared to the thoracic artery wall of normal diet mice and thoracic artery wall of high-fat diet administration group (rhHAPLN1), it may be seen that typical atherosclerotic lesions were distinct in the thoracic artery wall of the PBS group with the high-fat diet. In particular, it may be found that the elastic lamina in the middle and neointima has been degraded or destroyed. On the other hand, it was found that the thoracic artery wall in the same area of the group administered with human recombinant HAPLN1 protein was significantly restored in a shape almost similar to that of the normal-diet mice.

### 2-3. Identification of an elastin fiber improving effect in the artery wall of high-fat diet mice by rhHAPLN1

With the aging and disease on the artery wall, elastin fibers may gradually degrade and break down (Cardiovascular Research, Volume 110, Issue 3, 1 June 2016, Pages 298-308).

Accordingly, changes in the elastic lamina were observed using Verhoeff s Van Gieson/EVG (ab 150667, USA) staining method in a thoracic aorta sample of high-fat diet mice prepared as in 2-2. The aortic sample slides underwent a process of deparaffinization and rehydration and then was treated with elastin fiber staining reagents and differentiation reagents. After staining with sodium thiosulfate solution and Van Gieson solution, they were finally dehydrated in alcohol. Finally, it was fixed using Canada balsam (C1795, Sigma).

In order to maintain the accuracy in the measurement, the same area was selected and measured in the arterial staining area. Considering that the elastic lamina may vary depending on the cross-section of the aortic wall, the same cross-section at about 40% of the top to bottom was selected and the degradation and recovery of the elastic lamina was evaluated using a 400x magnification. Elastic lamina of mice fed with common food was used as a negative control group, and that of mice fed with high-fat diet was used as a positive control group.

In addition, the evaluation criteria for the elastin degradation stage have four stages as follows:
Stage 1: There is no elastin degradation, and the elastic layer fully exists in the tissue.
Stage 2: There is slight degradation or rupture in the lamina elastin.
Stage 3: there is severe degradation or multiple ruptures in the lamina elastin.
Stage 4: Elastin is seriously degraded or lost, or the aortic wall ruptures.

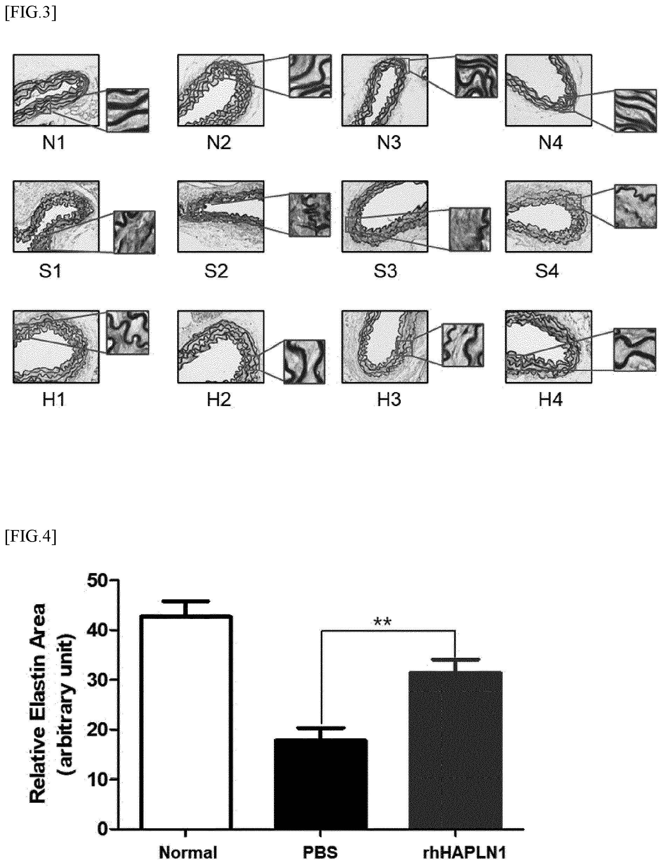

An elastin fiber recovery effect in the black area (elastin + area) of elastin fiber observed in FIG. 3 was determined using Image J software. Data were presented as mean ± SD (** P <0.01).

As a result, as shown in FIGS. 2 to 4, it was found that the rhHAPLN1 treated group inhibited the elastic lamina degraded by the high-fat diet statistically significantly compared to the control group.

### 2-4. Staining and quantitation of transgelin (SM22α) in the mouse artery wall

Quantitative changes in transgelin (SM22α), a key biomarker of atherosclerotic arterial vessel walls, were observed. To observe the change in transgelin levels in the group abdominally administered with rhHAPLN1 of each mouse (n=8) fed with a high-fat diet for 4 months, fluorescent substance-labeled red transgelin antibody (anti-transgelin antibody labeled with Alexa Fluor) was used for staining. In addition, the red area (transgelin + area) of a transgelin region was measured using Image J software.

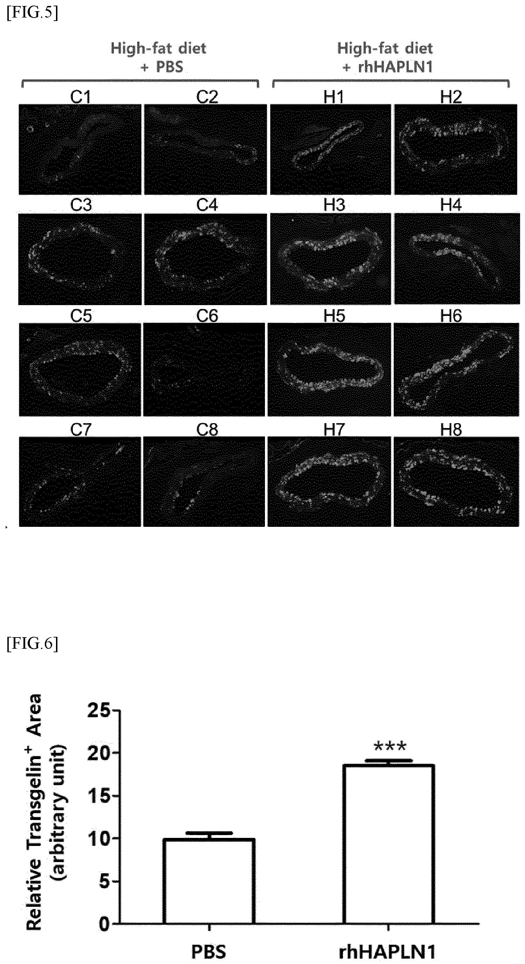

As a result, as shown in FIGS. 5 and 6, it was found that the area of transgelin increased in the rhHAPLN1 treated group.

### <Example 3> Identification of a signaling pathway changing effect by administration of recombinant human HAPLN1 protein (rhHAPLN1) into vascular smooth muscle cells (VSMCs)

### 3-1. Cell culture of human aortic smooth muscle cells (HASMCs; a type of VSMC)

Human aortic smooth muscle cells (304-05a, Cell Applications, USA) were cultured in HASMC growth media (# 311-500) using a T 75 flask. Cells were cultured in a humidified incubator in the presence of 5% CO₂ at 37°C. When HASMCs were about 80%-90% grown, they were subcultured. Cells with the passage count of 4-8 were used in the experiment.

### 3-2. Identification of an NF-kB phosphorylation inhibitory effect in HASMC

Inflammation and overproliferation of HASMC play a key role in a variety of vascular diseases, including restenosis, atherosclerosis, and hypertension. In cultured HASMC, the inflammatory and cytogenic cytokine IL-1β inhibits expression of the VSMC anti-atherosclerotic marker gene and induces the expression of inflammatory genes. Activation of NF-κB may promote the proliferation of HASMC and induce migration of HASMC, and other matrix metalloproteinases (MMPs) that cause extracellular matrix (ECM) damage and plaque rupture may be upregulated. Therefore, inhibition of the expression of NF-κB has important implications for the regulation of atherosclerosis.

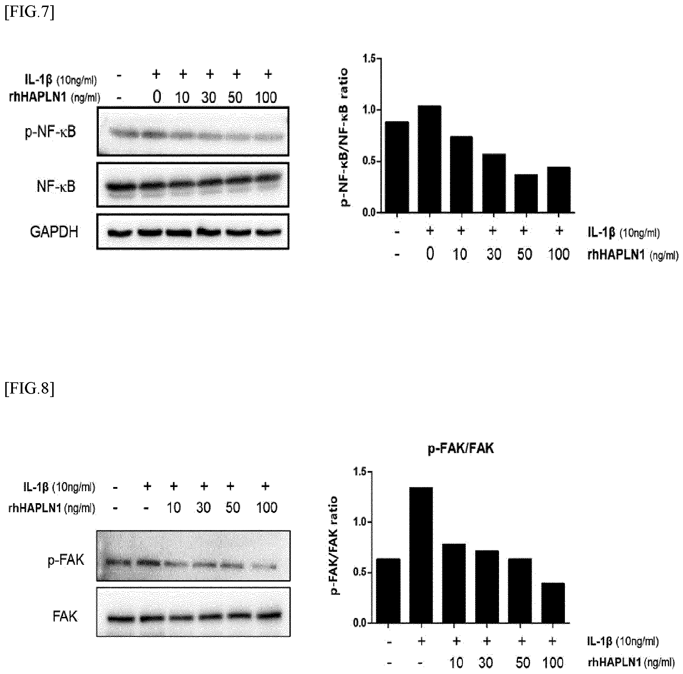

As a result of treating cultured HASMCs with interleukin-1beta (IL-1β), which is known to be an inducer of vascular aging to observe changes in the activation and phosphorylation of NF-κB, it was found that the phosphorylated NF-κB (p-NF-κB) increased by IL-1β was suppressed by recombinant human HAPLN1 protein (rhHAPLN1), as shown in FIG. 7. This indicates that NF-κB is not only related to inflammatory factors but may also affect proliferation of VSMCs.

### 3-3. Identification of a focal adhesion kinase (FAK) phosphorylation inhibitory effect in human aortic smooth muscle cells (HASMCs)

Excessive proliferation and migration of HASMC contributes to obstructive cardiovascular diseases such as atherosclerosis. HASMC in mature blood vessels shows inhibited proliferation and migration, but inflammatory stimulatory stimulation after vascular damage increases secretion of growth factors and matrix synthesis to promote the proliferation of HASMC and its migration to the intima of blood vessels, causing arteriosclerosis due to arteriostenosis.

Nuclear focal adhesion kinase (FAK) regulates proliferation of vascular smooth muscle cells (VSMCs) and proliferation of neointima via GATA4-mediated cyclin D1 transcription. FAK is a type of protein tyrosine kinases that mediate the integrins and growth factor signaling pathways involved in cell proliferation and migration, and is involved in the migration, proliferation, and inflammatory response of HASMCs. Therefore, inhibition of activation of FAK through phosphorylation means that the development of atherosclerosis may be inhibited.

As a result of treating cultured HASMCs with interleukin-1beta (IL-1β), which is known to be an inducer of vascular aging to observe changes in activation and phosphorylation of FAK, it was found that the phosphorylated FAK (p-FAK) increased by IL-1β was suppressed by the recombinant human HAPLN1 protein (rhHAPLN1), as shown in FIG. 8.

As described above, a specific part of the content of the present disclosure is described in detail, for those of ordinary skill in the art, it is clear that the specific description is only a preferred example embodiment, and the scope of the present disclosure is not limited thereby. In other words, the substantial scope of the present disclosure may be defined by the appended claims and their equivalents.

## Claims

1. A pharmaceutical composition for preventing or treating a vascular disease, comprising hyaluronan and proteoglycan link protein 1 (HAPLN1) as an active ingredient.

2. The pharmaceutical composition of claim 1, wherein the HAPLN1 is a protein fragment having an amino acid sequence represented by SEQ ID NO: 1.

3. The pharmaceutical composition of claim 1, wherein the composition inhibits and alleviates damage in elastic lamina of a vascular wall.

4. The pharmaceutical composition of claim 1, wherein the composition ameliorates loss of transgelin.

5. The pharmaceutical composition of claim 1, wherein the composition suppresses phosphorylation of NF-κB or focal adhesion kinase (FAK) in vascular smooth muscle cells (VSMCs).

6. The pharmaceutical composition of claim 1, wherein the vascular disease is one or more selected from the group consisting of arteriosclerosis, hypertension, aneurysm, hemorrhage, cerebral infarction due to vascular wall obstruction, cerebral hemorrhage, ischemic heart disease, myocardial infarction, and peripheral vascular disease.

7. A health functional food composition for preventing or alleviating a vascular disease, comprising hyaluronan and proteoglycan link protein 1 (HAPLN1) as an active ingredient.

8. The health functional food composition of claim 7, wherein the HAPLN1 is a protein fragment having an amino acid sequence represented by SEQ ID NO: 1.

9. The health functional food composition of claim 7, wherein the vascular disease is one or more selected from the group consisting of arteriosclerosis, hypertension, aneurysm, hemorrhage, cerebral infarction due to vascular wall obstruction, cerebral hemorrhage, ischemic heart disease, myocardial infarction, and peripheral vascular disease.
